(19) Europäisches Patentamt
European Patent Office
Office européen des brevets

(11) EP 1 243 322 A1

(12) EUROPÄISCHE PATENTANMELDUNG

(43) Veröffentlichungstag:
**25.09.2002 Patentblatt 2002/39**

(51) Int Cl.7: **B01J 13/02**

(21) Anmeldenummer: **01107153.7**

(22) Anmeldetag: **22.03.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Primacare S.L., c/o Cognis Iberica S.L.
08040 Barcelona (ES)**

(72) Erfinder:
- **Viladot Petit, Josep-Lluis
08018 Barcelona (ES)**
- **De Moragas, Maria
08310 Argentona (Barcelona) (ES)**

(74) Vertreter: **Fabry, Bernd, Dr.
c/o Cognis Deutschland GmbH,
Dep. Intellectual Properties,
Postfach 130164
40551 Düsseldorf (DE)**

(54) **Mikrokapseln(X)**

(57) Vorgeschlagen werden Mikrokapseln mit mittleren Durchmessern im Bereich von 0,1 bis 5 mm, bestehend aus einer Hüllmembran und einer mindestens einen Wirkstoff enthaltenden Matrix, dadurch erhältlich, dass man

(a1) aus Gelbildnern, Chitosanen und Wirkstoffen eine Matrix herstellt,
(a2) die Matrix in einer Ölphase dispergiert,
(a3) zur Bildung der Hülle die dispergierte Matrix mit wässrigen Lösungen anionischer Tenside in Kontakt bringt, und
(a4) die Ölphase wieder entfernt,

oder

(b1) aus Gelbildnern, anionischen Tensiden und Wirkstoffen eine Matrix herstellt,
(b2) die Matrix in einer Ölphase dispergiert,
(b3) zur Bildung der Hülle die dispergierte Matrix mit wässrigen Chitosanlösungen in Kontakt bringt, und
(b4) die Ölphase wieder entfernt.

EP 1 243 322 A1

## Beschreibung

### Gebiet der Erfindung

Die Erfindung befindet sich auf den Gebieten der Kosmetik bzw. Pharmazie sowie der Textilausrüstungsmittel und betrifft neue Mikrokapseln, ein Verfahren zu deren Herstellung sowie deren Verwendung in der Kosmetik und Textiltechnik.

### Stand der Technik

Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgieirung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

In diesem Zusammenhang sei auch auf die deutsche Patentanmeldung **DE 19712978 A1** (Henkel) hingewiesen, aus der Chitosanmikrosphären bekannt sind, die man erhält, indem man Chitosane oder Chitosanderivate mit Ölkörpern vermischt und diese Mischungen in alkalisch eingestellte Tensidlösungen einbringt. Aus der deutschen Patentanmeldung **DE 19756452 A1** (Henkel) ist ferner auch die Verwendung von Chitosan als Verkapselungsmaterial für Tocopherol bekannt. Chitosanmikrokapseln und Verfahren zu ihrer Herstellung sind Gegenstand früherer Patenanmeldungen der Patentanmelderin **[WO 01/01926, WO 01/01927, WO 01/01928, WO 01/01929].** Man unterscheidet dabei im wesentlichen die beiden folgenden Verfahren:

(1) Mikrokapseln mit mittleren Durchmessern im Bereich von 0,1 bis 5 mm, bestehend aus einer Hüllmembran und einer mindestens ein Wachs enthaltenden Matrix, erhältlich, indem man

(a) aus Gelbildnern, Chitosanen und Wirkstoffen eine Matrix zubereitet,
(b) gegebenenfalls die Matrix in einer Ölphase dispergiert,
(c) die dispergierte Matrix mit wässrigen Lösungen anionischer Polymeren vom Typ der Polyalginate oder anionischen Chitosanderivate behandelt und gegebenenfalls dabei die Ölphase entfernt.

(2) Mikrokapseln mit mittleren Durchmessern im Bereich von 0,1 bis 5 mm, bestehend aus einer Hüllmembran und einer mindestens ein Wachs enthaltenden Matrix, erhältlich, indem man

(a) aus Gelbildnern, anionischen Polymeren vom Typ der Polyalginate oder anionischen Chitosanderivate und Wirkstoffen eine Matrix zubereitet,
(b) gegebenenfalls die Matrix in einer Ölphase dispergiert,
(c) die dispergierte Matrix mit wässrigen Chitosanlösungen behandelt und gegebenenfalls dabei die Ölphase entfernt.

Die Mikrokapseln des Stands der Technik sind jedoch nicht in jeder Hinsicht zufriedenstellend. Insbesondere wird als negativ empfunden, dass die Kapseln zu wenig Schaum entwickeln, was insbesondere bei der Flammschutzausrüstung als negativ empfunden wird. Die Aufgabe der vorliegenden Erfindung hat somit darin bestanden, Mikrokapseln mit den bekannten vorteilhaften Eigenschaften zur Verfügung zu stellen, die jedoch bei der Freisetzung des Wirkstoffs eine größere Menge Schaum entwickeln.

## Beschreibung der Erfindung

Gegenstand der Erfindung sind Mikrokapseln mit mittleren Durchmessern im Bereich von 0,1 bis 5 mm, bestehend aus einer Hüllmembran und einer mindestens einen Wirkstoff enthaltenden Matrix, die man erhält, indem man

(a1) aus Gelbildnern, Chitosanen und Wirkstoffen eine Matrix herstellt,
(a2) die Matrix in einer Ölphase dispergiert,
(a3) zur Bildung der Hülle die dispergierte Matrix mit wässrigen Lösungen anionischer Tenside in Kontakt bringt, und
(a4) die Ölphase wieder entfernt,

oder

(b1) aus Gelbildnern, anionischen Tensiden und Wirkstoffen eine Matrix herstellt,
(b2) die Matrix in einer Ölphase dispergiert,
(b3) zur Bildung der Hülle die dispergierte Matrix mit wässrigen Chitosanlösungen in Kontakt bringt, und
(b4) die Ölphase wieder entfernt.

Überraschenderweise wurde gefunden, dass der Austausch von Polyalginaten bzw. anionischen Chitosanen gegen anionische Tenside gelingt und zu Mikrokapseln führt, die über verbesserte Schaumeigenschaften verfügen.
Ein weiterer Gegenstand der Erfindung betrifft ein Verfahren zur Herstellung von Mikrokapseln mit mittleren Durchmessern im Bereich von 0,1 bis 5 mm, bestehend aus einer Hüllmembran und einer mindestens einen Wirkstoff enthaltenden Matrix, bei dem man

(a1) aus Gelbildnern, Chitosanen und Wirkstoffen eine Matrix herstellt,
(a2) die Matrix in einer Ölphase dispergiert,
(a3) zur Bildung der Hülle die dispergierte Matrix mit wässrigen Lösungen anionischer Tenside in Kontakt bringt, und
(a4) die Ölphase wieder entfernt,
oder

(b1) aus Gelbildnern, anionischen Tensiden und Wirkstoffen eine Matrix herstellt,
(b2) die Matrix in einer Ölphase dispergiert,
(b3) zur Bildung der Hülle die dispergierte Matrix mit wässrigen Chitosanlösungen in Kontakt bringt, und
(b4) die Ölphase wieder entfernt.

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

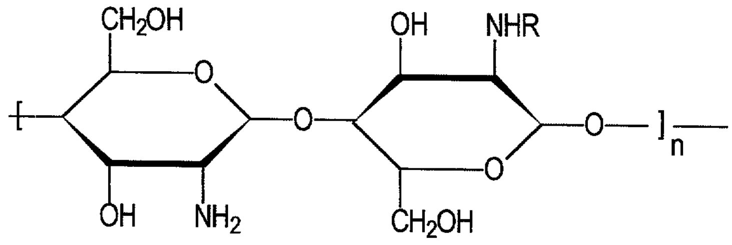

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-232).** Übersichten zu diesem Thema sind auch beispielsweise von B. Gesslein et al. in **HAPPI 27, 57 (1990),** O. Skaugrud in **Drug Cosm.Ind. 148, 24 (1991)** und E. Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177, 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

Wirkstoffe

Die Auswahl der Wirkstoffe, die in den neuen Mikrokapseln eingeschlossen sind, ist an sich unkritisch. Vorzugsweise handelt es sich um Stoffe, die erst durch mechanische Zerstörung der Mikrokapseln freigesetzt werden. In diesen Fällen kommt den Mikrokapseln die Aufgabe zu, den Kontakt zwischen äußerer Umgebung und Wirkstoff und damit eine chemische Reaktion bzw. einen Abbau zu verhindern. Es kann es sein, dass die in der Kapsel eingeschlossenen Stoffe überhaupt nicht freigesetzt werden sollen und ausschließlich dem Zweck dienen, der Zubereitung ein ästhetisches Äußeres zu verleihen; dies trifft beispielsweise vielfach für Farbstoffe zu. Es ist natürlich klar, dass diese Einsatzformen auch nebeneinander bestehen können. Insbesondere ist es möglich, beispielsweise einen Duftstoff für die spätere Freisetzung zusammen mit einem Farbpigment zu verkapseln, welches der Kapsel ein besonderes Aussehen verleiht.

Wirkstoffe für kosmetische oder pharmazeutische Anwendungen

Typische Beispiele für Wirkstoffe, wie sie im Bereich der kosmetischen und pharmazeutischen Zubereitungen eingesetzt werden sind kosmetische Öle, Perlglanzwachse, Stabilisatoren, biogene Wirkstoffe, Vitamine, Deodorantien, Antitranspirantien, Antischuppenmittel, UV-Lichtschutzfaktoren, Antioxidantien, Konservierungsmittel, Insektenrepellentien, Selbstbräuner, Typrosininhibitoren (Depigmentierungsmittel), Parfümöle, Aromen und Farbstoffe.

➤ Kosmetische Öle

Als kosmetische Öle kommen beispielsweise Guerbetalkohole auf Basis von Fettalkoholen mit 6 bis 18, vorzugsweise 8 bis 10 Kohlenstoffatomen, Ester von linearen $C_6$-$C_{22}$-Fettsäuren mit linearen $C_6$-$C_{22}$-Fettalkoholen,

Ester von verzweigten $C_6$-$C_{13}$-Carbonsäuren mit linearen $C_6$-$C_{22}$-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen $C_6$-$C_{22}$-Fettsäuren mit verzweigten Alkoholen, insbesondere 2-Ethylhexanol, Ester von Hydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis $C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, Guerbetcarbonate, Ester der Benzoesäure mit linearen und/oder verzweigten $C_6$-$C_{22}$-Alkoholen (z. B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

➢ Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

➢ Stabilisatoren

Als Stabilisatoren können Metallsalze von Fettsäuren, wie z.B. Magnesium-, Aluminiumund/oder Zinkstearat bzw. -ricinoleat eingesetzt werden.

➢ Biogene Wirkstoffe

Unter biogenen Wirkstoffen sind beispielsweise Tocopherol, Tocopherolacetat, Tocopherolpalmitat, Ascorbinsäure, Desoxyribonucleinsäure, Retinol, Retinylpalmitat, Bisabolol, Allantoin, Phytantriol, Panthenol, Menthol, Teebaumöl, AHA-Säuren, Kojisäure, Aminosäuren, Ceramide, Pseudoceramide, essentielle Öle, Pflanzenextrakte und Vitaminkomplexe zu verstehen.

➢ Deodorantien und Antischuppenmittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Tridosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-ndecylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, $\alpha$-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, $\beta$-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B.entzündungshemmende, hautschützende oder wohlriechende ätherische Öle, synthetische hautschützende Wirkstoffe und/oder öllösliche Parfümöle sein.

Als Antischuppenmittel können Climbazol, Octopirox, Ketokonazol und Zinkpyrethion eingesetzt werden.

➤ <u>UV-Lichtschutzfaktoren und Antioxidantien</u>

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen : 3-Benzylidencampher bzw. 3-Benzylidennorcampher und dessen Derivate, z.B. 3-(4-Methylbenzyliden)campher wie in der EP 0693471 B1 beschrieben; 4-Aminobenzoesäurederivate, vorzugsweise 4-(Dimethylamino)benzoesäure-2-ethylhexyl-ester, 4-Dimethylamino)-benzoesäure-2-octylester und 4-(Dimethylamino)benzoesäure-amylester; Ester der Zimtsäure, vorzugsweise 4-Methoxyzimtsäure-2-ethylhexylester, 4-Methoxyzimtsäurepropyl-ester, 4-Methoxyzimtsäureisoamylester 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethyl-hexylester; Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben.

Als wasserlösliche Substanzen kommen in Frage: 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze. Als typische UV-A-Filter kommen insbesondere Derivate des Benzoylmethans in Frage, wie beispielsweise 1-(4'tert.Butyl-phenyl)-3-(4'-methoxyphenyl)propan-1,3-dion, 4-tert.-Butyl-4'-methoxydibenzoyl-methan (Parsol 1789), 1-Phenyl-3-(4'-isopropylphenyl)-propan-1,3-dion sowie Enaminverbindungen, wie beschrieben in der DE 19712033 A1 (BASF). Die UV-A und UVB-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden als verkapselte Wirkstoffe bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl- , Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-Eacetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, $ZnSO_4$) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

- Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insektenrepellentien kommen N,N-Diethyl-m-toluamid, 1,2-Pentandiol oder Ethyl Butylacetylaminopropionate in Frage, als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

- Parfümöle und Farbstoffe

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Wirkstoffe können auch ausschließlich aus ästhetischen Gründen in den Kapseln enthalten und nicht für eine kontrollierte Freigabe vorgesehen sein.

Wirkstoffe für Anwendungen im Textilbereich

Ein zweiter Einsatzbereich der neuen Mikrokapseln besteht in der Ausrüstung von Textilien. Der besondere Vorteil liegt hier darin, dass die Kapseln bei deutlich höheren Temperaturen erweichen und das Flammschutzmittel nicht zu früh freisetzen. Typische Beispiele für geeignete Wirkstoffe sind alle Arten von Flammschutzmitteln, also Metalloxide, wie z.B. Magnesiumoxid, Calciumoxid, Aluminiumoxid oder Antimon(III)oxid, organische Halide, wie z.B. Tetrabrombisphenol A oder Decabromodiphenyloxid sowie insbesondere organische Phosphorverbindungen, wie z. B. Triphenylphosphat (TPP), Tricresylphosphat (TCP), Cresyldiphenylphosphat (CDP) oder Tetraphenyldiphosphat. Die Mittel werden vorzugsweise mit Teilchengrößen im Bereich von 0,1 bis 5 mm eingesetzt.

Die Wirkstoffe können dabei in solchen Mengen eingesetzt werden, dass sich in den Mikrokapseln ein Gehalt von 0,1 bis 5, vorzugsweise 0,5 bis 3 und insbesondere 1 bis 2 Gew.-% ergibt.

Tensidische Emulgatoren

In einer bevorzugten Ausführungsform der vorliegenden Erfindung werden die Gelbildner, Chitosane und Wirkstoffe gemeinsam mit nichtionischen Emulgatoren eingesetzt. Als Emulgatoren kommen beispielsweise nichtionogene Tenside aus mindestens einer der folgenden Gruppen in Frage:

- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren

mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;

- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

- Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. $C_{12/18}$-Fettsäuremono- und -diester von Anlagerungsprodukten von Ethylenoxid an Glycerin sind aus **DE 2024051 PS** als Rückfettungsmittel für kosmetische Zubereitungen bekannt.

- Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

- Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

- Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego

Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Üblicherweise werden die Emulgatoren in Mengen von 1 bis 15, vorzugsweise 2 bis 10 und insbesondere 8 bis 10 Gew.-% - bezogen auf die Summe von Gelbildnern, Chitosanen bzw. anionischen Tensiden und Wirkstoffen - eingesetzt.

Ölphase

Als Ölphase, in der die Matrix feindispergiert wird, kommen die bereits im Kapitel "Wirkstoffe" genannten kosmetischen Ölkörper in Frage. Vorzugsweise arbeitet man in Paraffin- oder Pflanzenölen, wobei die Ölphase üblicherweise das 2 bis 5fache des Volumens der Matrix beträgt.

Anionische Tenside

Die anionische Tenside haben die Aufgabe, mit den Chitosanen Membranen zu bilden. Für diesen Zweck eignen sich beispielsweise Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Vorzugsweise werden Alkylbenzolsulfonate, Alkyl(ether)sulfate, Alkyl(ether)phosphate oder Seifen sowie deren Gemische eingesetzt. Bevorzugte Alkylbenzolsulfonate folgen der Formel **(I),**

$$R^1\text{-Ph-}SO_3X \qquad \text{(I)}$$

in der $R^1$ für einen verzweigten, vorzugsweise jedoch linearen Alkylrest mit 10 bis 18 Kohlenstoffatomen, Ph für einen Phenylrest und X für ein Alkali- und/oder Erdalkalimetall, Ammonium, Alkylammonium, Alkanolammonium oder Glucammonium steht. Insbesondere von diesen geeignet sind Dodecylbenzolsulfonate, Tetradecylbenzolsulfonate, Hexadecylbenzolsulfonate sowie deren technische Gemische in Form der Natriumsalze.

Unter Alkyl- und/oder Alkenyl(ether)sulfaten, die auch häufig als Fettalkoholsulfate bzw. -ethersulfate bezeichnet werden, sind die Sulfatierungsprodukte primärer und/oder sekundärer Alkohole oder deren Ethylenoxidaddukten zu verstehen, die vorzugsweise der Formel **(II)** folgen,

$$R^2O(CH_2CH_2O)_nSO_3X \qquad \text{(II)}$$

in der $R^2$ für einen linearen oder verzweigten, aliphatischen Alkyl- und/oder Alkenylrest mit 6 bis 22, vorzugsweise 12 bis 18 Kohlenstoffatomen, n für 0 oder Zahlen von 1 bis 10 und X für ein Alkali- und/oder Erdalkalimetall, Ammonium, Alkylammonium, Alkanolammonium oder Glucammonium steht. Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelenschen Oxosynthese erhalten werden. Typische Beispiele für Alkylethersulfate sind die Sulfatierungsprodukte der Addukte der genannten Alkohole mit 1 bis 10 Mol Ethylenoxid. Die Sulfatierungsprodukte können vorzugsweise in Form ihrer Alkalisalze und insbesondere ihrer Natriumsalze eingesetzt werden. Besonders bevorzugt sind Alkyl(ether)sulfate auf Basis von $C_{16/18}$-Talg-Fettalkoholen bzw. pflanzlichen Fettalkoholen vergleichbarer

C-Kettenverteilung in Form ihrer Natriumsalze. Im Falle von verzweigten primären Alkoholen handelt es sich um Oxoalkohole, wie sie z.B. durch Umsetzung von Kohlenmonoxid und Wasserstoff an alpha-ständige Olefine nach dem Shop-Verfahren zugänglich sind. Solche Alkoholmischungen sind im Handel unter dem Handelsnamen Dobanol® oder Neodol® erhältlich. Geeignete Alkoholmischungen sind Dobanol 91®, 23®, 25®, 45®. Eine weitere Möglichkeit sind Oxoalkohole, wie sie nach dem klassischen Oxoprozess der Enichema bzw. der Condea durch Anlagerung von Kohlenmonoxid und Wasserstoff an Olefine erhalten werden. Bei diesen Alkoholmischungen handelt es sich um eine Mischung aus stark verzweigten Alkoholen. Solche Alkoholmischungen sind im Handel unter dem Handelsnamen Lial® erhältlich. Geeignete Alkoholmischungen sind Lial 91®, 111®, 123®, 125®, 145®.

Unter Alkyl(ether)phosphaten sind anionische Tenside zu verstehen, die durch Phosphatierung von Fettalkoholen oder deren Ethylenoxidaddukten erhalten werden und vorzugsweise der Formel **(III)** folgen,

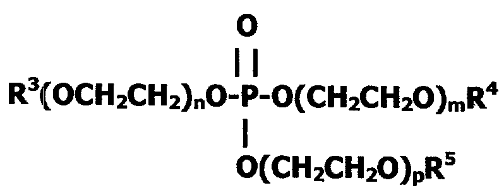

**(III)**

in der $R^3$ für einen linearen oder verzweigten Alkyl- und/oder Alkenylrest mit 6 bis 22 Kohlenstoffatomen, $R^4$ und $R^5$ unabhängig voneinander für Wasserstoff, ein Alkalimetall oder $R^3$, die Summe (n+m+p) für 0 oder Zahlen von 1 bis 10 steht. Typische Beispiele sind Mono-, Di- und Triphosphate sowie deren technische Gemische auf Basis von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen und Addukten mit 1 bis 10 Mol Ethylenoxid.

Unter Seifen sind weiterhin Fettsäuresalze der Formel **(IV)** zu verstehen,

$$R^6CO\text{-}OX \qquad (IV)$$

in der $R^6CO$ für einen linearen oder verzweigten, gesättigten oder ungesättigten Acylrest mit 6 bis 22 und vorzugsweise 12 bis 18 Kohlenstoffatomen und wiederum X für Alkali- und/oder Erdalkali, Ammonium, Alkylammonium oder Alkanolammonium steht. Typische Beispiele sind die Natrium-, Kalium-, Magnesium-, Ammonium- und Triethanolammoniumsalze der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Kokos- oder Palmkernfettsäure in Form ihrer Natrium- oder Kaliumsalze eingesetzt.

<u>Herstellverfahren</u>

Zur Herstellung der neuen Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und den Wirkstoff in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Chitosan und Wirkstoff wird die Matrix in einer Ölphase unter starker Scherung sehr fein dispergiert, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im dritten Schritt erfolgt dann die eigentliche Verkapselung, d.h. die Ausbildung der Hüllmembran durch Inkontaktbringen des Chitosans

in der Matrix mit den anionischen Tensiden. Hierzu empfiehlt es sich, die in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%igen wässrigen Lösung des anorganischen Phosphats zu waschen und dabei gleichzeitig die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise 1 bis 3 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die anionischen Tenside auch zur Herstellung der Matrix einsetzen und die Verkapselung mit den Chitosanen durchführen.

**Gewerbliche Anwendbarkeit**

Weitere Gegenstände der Erfindung betreffen die Verwendung der Mikrokapseln zur Herstellung von kosmetischen bzw. pharmazeutischen Zubereitungen sowie Garnen, Fasern oder textilen Flächengebilden, die Kapseln in Mengen von 0,1 bis 15, vorzugsweise 1 bis 10 und insbesondere 3 bis 8 Gew.-% enthalten können.

Kosmetische und/oder pharmazeutische Zubereitungen

Die erfindungsgemäßen Mikrokapseln können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholischen und wässrig/alkoholischen Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Die entsprechenden Zusatzstoffe sind bereits in den Kapiteln Wirkstoffe/tensidische Emulgatoren erläutert worden, so dass auf eine Wiederholung an dieser Stelle verzichtet werden kann.

Textilien

Die erfindungsgemäßen Mikrokapseln können analog zum Einsatz bei natürlichen Keratinfasern auch zur Ausrüstung von Textilien dienen, obschon hier der Schwerpunkt in der Verbesserung des Flammschutzes liegt. In gleicher Weise können die Garne ausgerüstet werden, indem man die Nanokapseln beispielsweise als Bestandteile von Spulölen oder Spinnfaserpräparationen aufbringt. Demzufolge eignen sich die Mikrokapseln zur Ausrüstung aller textiler Flächengebilde. In einer besonderen Ausführungsform der vorliegenden Erfindung enthalten die Nanokapseln neben den Flammschutzmitteln noch fettlösliche Farbstoffe, wie beispielsweise Beta-Carotine oder Tocopherole, die dann als Indikatoren dafür dienen können, dass die Kapseln vom Gewebe aufgenommen worden sind.

**Beispiele**

**Beispiel 1.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g Paraffinöl, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 2.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g Squalan, 0,5 g Phenonip® und 0,5 g Ceteareth-20 in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 3.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g Panthenol, 0,5 g Phenonip® und 3 g Cetearylglucosid in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 4.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 75 g Wasser und dann mit einer Zubereitung von 50 g einer 50 Gew.-%igen Sodium Laureth Sulfate-Paste, 10 g Panthenol, 0,5 g Phenonip® und 3 g Cetearylglucosid in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit 1 Gew.-%igen Chitosanlösung (Hydagen® DCMF, Cognis Deutschland GmbH, Düsseldorf/FRG) und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 5.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g β-Carotin, 0,5 g Phenonip® und 3 g Cetearylglucosid in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 6.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g Tocopherolacetat, 0,5 g Phenonip® und 3 g Cetearylglucosid in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 7.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 10 g Ascorbinsäure, 0,5 g Phenonip® und 3 g Ceteareth-12 in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Laureth Sulfate und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 8.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren

zunächst mit einer homogenen Dispersionen von 10 g Glycerin und 2 g Eisen(II)oxid in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG) und 10 g Tricresylphosphat (TCP) in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Lauryl Sulfate und dann mehrfach gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 9.** In einem 500-ml-Drelhalskolben mit Rührer und Rückflusskühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Antimon(III)oxid in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), Cresyldiphenylphosphat (CDP) ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Lauryl Sulfate und dann mehrfach gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

**Beispiel 10.** In einem 500-ml-Dreihalskolben mit Rührer und Rückflusskühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis Deutschland GmbH, Düsseldorf/FRG), 5 g Aluminiumhydroxid und 5 g Magnesiumhydroxid in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Lösung von Sodium Dodecyl Benzenesulfonate und dann mehrfach gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

Formulierungsbeispiele sind der nachstehenden Tabelle 1 zu entnehmen.

**Tabelle 1**

**Beispiele für kosmetische Zubereitungen (Wasser, Konservierungsmittel ad 100 Gew.-%)**

| Zusammensetzung (INCI) | 1 | 2 | 3 | 4 | 5 | 6 | 7 | 8 | 9 | 10 |
|---|---|---|---|---|---|---|---|---|---|---|
| **Texapon® NSO**<br>Sodium Laureth Sulfate | - | - | - | - | - | - | 38,0 | 38,0 | 25,0 | - |
| **Texapon® SB 3**<br>Disodium Laureth Sulfosuccinate | - | - | - | - | - | - | - | - | 10,0 | - |
| **Plantacare® 818**<br>Coco Glucosides | - | - | - | - | - | - | 7,0 | 7,0 | 6,0 | - |
| **Plantacare® PS 10**<br>Sodium Laureth Sulfate (and) Coco Glucosides | - | - | - | - | - | - | - | - | - | 16,0 |
| **Dehyton® PK 45**<br>Cocamidopropyl Betaine | - | - | - | - | - | - | - | - | 10,0 | - |
| **Dehyquart® A**<br>Cetrimonium Chloride | 2,0 | 2,0 | 2,0 | 2,0 | 4,0 | 4,0 | - | - | - | - |
| **Dehyquart L® 80**<br>Dicocoylmethylethoxymonium Methosulfate (and) Propylenglycol | 1,2 | 1,2 | 1,2 | 1,2 | 0,6 | 0,6 | - | - | - | - |
| **Eumulgin® B2**<br>Ceteareth-20 | 0,8 | 0,8 | - | 0,8 | - | 1,0 | - | - | - | - |
| **Eumulgin® VL 75**<br>Lauryl Glucoside (and) Polyglyceryl-2 Polyhydroxystearate (and) Glycerin | - | - | 0,8 | - | 0.8 | - | - | - | - | - |
| **Lanette® O**<br>Cetearyl Alcohol | 2,5 | 2,5 | 2,5 | 2,5 | 3,0 | 2,5 | - | - | - | - |
| **Cutina® GMS**<br>Glyceryl Stearate | 0,5 | 0,5 | 0,5 | 0,5 | 0,5 | 1,0 | - | - | - | - |
| **Cetiol® HE**<br>PEG-7 Glyceryl Cocoate | 1,0 | - | - | - | - | - | - | - | 1,0 | |
| **Cetiol® PGL**<br>Hexyldecanol (and) Hexyldecyl Laurate | - | 1,0 | - | - | 1,0 | - | - | - | - | - |
| **Cetiol® V**<br>Decyl Oleate | - | - | - | 1,0 | - | - | - | - | - | - |
| **Eutanol® G**<br>Octyldodecanol | - | - | 1,0 | - | - | 1,0 | - | - | - | - |
| **Nutrilan® Keratin W**<br>Hydrolyzed Keratin | - | - | - | 2,0 | - | - | - | - | - | - |
| **Lamesoft® LMG**<br>Glyceryl Laurate (and) Potassium Cocoyl Hydrolyzed Collagen | - | - | - | - | - | - | 3,0 | 2,0 | 4,0 | - |
| **Euperlan® PK 3000 AM**<br>Glycol Distearate (and) Laureth-4 (and) Cocamidopropyl Betaine | - | - | - | - | - | - | - | 3,0 | 5,0 | 5,0 |
| **Generol® 122 N**<br>Soja Sterol | - | - | - | - | 1,0 | 1,0 | - | - | - | - |
| **Panthenol-Mikrokapseln gemäß Bsp.3** | 1,0 | 1,0 | 1,0 | 1,0 | 1,0 | 1,0 | 1,0 | 1,0 | 1,0 | 1,0 |
| **Hydagen® CMF**<br>Chitosan | 1,0 | 1,0 | 1,0 | 1,0 | 1,0 | 1,0 | 1,0 | 1,0 | 1,0 | 1,0 |
| **Copherol® 1250**<br>Tocopherol Acetate | - | - | 0,1 | 0,1 | - | - | - | - | - | - |
| **Arlypon® F**<br>Laureth-2 | - | - | - | - | - | - | 3,0 | 3,0 | 1,0 | - |
| **Sodium Chloride** | - | - | - | - | - | - | - | 1,5 | - | 1,5 |

(1-4) Haarspülung, (5-6) Haarkur, (7-8) Duschbad, (9) Duschgel, (10) Waschlotion

**Tabelle 1**
**Beispiele für kosmetische Zubereitungen (Wasser, Konservierungsmittel ad 100 Gew.-%) – Forts.**

| Zusammensetzung (INCI) | 11 | 12 | 13 | 14 | 15 | 16 | 17 | 18 | 19 | 20 |
|---|---|---|---|---|---|---|---|---|---|---|
| **Texapon® NSO**<br>Sodium Laureth Sulfate | 20,0 | 20,0 | 12,4 | - | 25,0 | 11,0 | - | - | - | - |
| **Texapon® K 14 S**<br>Sodium Myreth Sulfate | - | - | - | - | - | - | - | - | 11,0 | 23,0 |
| **Texapon® SB 3**<br>Disodium Laureth Sulfosuccinate | - | - | - | - | - | 7,0 | - | - | - | - |
| **Plantacare® 818**<br>Coco Glucosides | 5,0 | 5,0 | 4,0 | - | - | - | - | - | 6,0 | 4,0 |
| **Plantacare® 2000**<br>Decyl Glucoside | - | - | - | - | 5,0 | 4,0 | - | - | - | - |
| **Plantacare® PS 10**<br>Sodium Laureth Sulfate (and) Coco Glucosides | - | - | - | 40,0 | - | - | 16,0 | 17,0 | - | - |
| **Dehyton® PK 45**<br>Cocamidopropyl Betaine | 20,0 | 20,0 | - | - | 8,0 | - | - | - | - | 7,0 |
| **Eumulgin® B1**<br>Ceteareth-12 | - | - | - | - | 1,0 | - | - | - | - | - |
| **Eumulgin® B2**<br>Ceteareth-20 | - | - | - | 1,0 | - | - | - | - | - | - |
| **Lameform® TGI**<br>Polyglyceryl-3 Isostearate | - | - | - | 4,0 | - | - | - | - | - | - |
| **Dehymuls® PGPH**<br>Polyglyceryl-2 Dipolyhydroxystearate | - | - | 1,0 | - | - | - | - | - | - | - |
| **Monomuls® 90-L 12**<br>Glyceryl Laurate | - | - | - | - | - | - | - | - | 1,0 | 1,0 |
| **Cetiol® HE**<br>PEG-7 Glyceryl Cocoate | - | 0,2 | - | - | - | - | - | - | - | - |
| **Eutanol® G**<br>Octyldodecanol | - | - | - | 3,0 | - | - | - | - | - | - |
| **Nutrilan® Keratin W**<br>Hydrolyzed Keratin | - | - | - | - | - | - | - | - | 2,0 | 2,0 |
| **Nutrilan® I**<br>Hydrolyzed Collagen | 1,0 | - | - | - | - | 2,0 | - | 2,0 | - | - |
| **Lamesoft® LMG**<br>Glyceryl Laurate (and) Potassium Cocoyl Hydrolyzed Collagen | - | - | - | - | - | - | - | - | 1,0 | - |
| **Lamesoft® 156**<br>Hydrogenated Tallow Gyceride (and) Potassium Cocoyl Hydrolyzed Collagen | - | - | - | - | - | - | - | - | - | 5,0 |
| **Gluadin® WK**<br>Sodium Cocoyl Hydrolyzed Wheat Protein | 1,0 | 1,5 | 4,0 | 1,0 | 3,0 | 1,0 | 2,0 | 2,0 | 2,0 | - |
| **Euperlan® PK 3000 AM**<br>Glycol Distearate (and) Laureth-4 (and) Cocamidopropyl Betaine | 5,0 | 3,0 | 4,0 | - | - | - | - | 3,0 | 3,0 | - |
| **Arlypon® F**<br>Laureth-2 | 2,6 | 1,6 | - | 1,0 | 1,5 | - | - | - | - | - |
| **Panthenol-Mikrokapseln gemäß Bsp.3** | 1,0 | 1,0 | 1,0 | 1,0 | 1,0 | 1,0 | 1,0 | 1,0 | 1,0 | 1,0 |
| **Hydagen® CMF**<br>Chitosan | 1,0 | 1,0 | 1,0 | 1,0 | 1,0 | 1,0 | 1,0 | 1,0 | 1,0 | 1,0 |
| **Sodium Chloride** | - | - | - | - | - | 1,6 | 2,0 | 2,2 | - | 3,0 |
| **Glycerin** (86 Gew.-%ig) | - | 5,0 | - | - | - | - | - | 1,0 | 3,0 | - |

(11-14) Duschbad „Two-in-One), (15-20) Shampoo

**Tabelle 1**
**Beispiele für kosmetische Zubereitungen (Wasser, Konservierungsmittel ad 100 Gew.-%) – Forts.**

| Zusammensetzung (INCI) | 21 | 22 | 23 | 24 | 25 | 26 | 27 | 28 | 29 | 30 |
|---|---|---|---|---|---|---|---|---|---|---|
| **Texapon® NSO**<br>Sodium Laureth Sulfate | - | 30,0 | 30,0 | - | 25,0 | - | - | - | - | - |
| **Plantacare® 818**<br>Coco Glucosides | - | 10,0 | - | - | 20,0 | - | - | - | - | - |
| **Plantacare® PS 10**<br>Sodium Laureth Sulfate (and) Coco Glucosides | 22,0 | - | 5,0 | 22,0 | - | - | - | - | - | - |
| **Dehyton® PK 45**<br>Cocamidopropyl Betaine | 15,0 | 10,0 | 15,0 | 15,0 | 20,0 | - | - | - | - | - |
| **Emulgade® SE**<br>Glyceryl Sterate (and) Ceteareth 12/20 (and) Cetearyl Alcohol (and) Cetyl Palmitate | - | - | - | - | - | 5,0 | 5,0 | 4,0 | - | - |
| **Eumulgin® B1**<br>Ceteareth-12 | - | - | - | - | - | - | - | 1,0 | - | - |
| **Lameform® TGI**<br>Polyglyceryl-3 Isostearate | - | - | - | - | - | - | - | - | 4,0 | - |
| **Dehymuls® PGPH**<br>Polyglyceryl-2 Dipolyhydroxystearate | - | - | - | - | - | - | - | - | - | 4,0 |
| **Monomuls® 90-O 18**<br>Glyceryl Oleate | - | - | - | - | - | - | - | - | 2,0 | - |
| **Cetiol® HE**<br>PEG-7 Glyceryl Cocoate | 2,0 | - | - | 2,0 | 5,0 | - | - | - | - | 2,0 |
| **Cetiol® OE**<br>Dicaprylyl Ether | - | - | - | - | - | - | - | - | 5,0 | 6,0 |
| **Cetiol® PGL**<br>Hexyldecanol (and) Hexyldecyl Laurate | - | - | - | - | - | - | - | 3,0 | 10,0 | 9,0 |
| **Cetiol® SN**<br>Cetearyl Isononanoate | - | - | - | - | - | 3,0 | 3,0 | - | - | - |
| **Cetiol® V**<br>Decyl Oleate | - | - | - | - | - | 3,0 | 3,0 | - | - | - |
| **Myritol® 318**<br>Coco Caprylate Caprate | - | - | - | - | - | - | - | 3,0 | 5,0 | 5,0 |
| **Bees Wax** | - | - | - | - | - | - | - | - | 7,0 | 5,0 |
| **Nutrilan® Elastin E20**<br>Hydrolyzed Elastin | - | - | - | - | - | 2,0 | - | - | - | - |
| **Nutrilan® I-50**<br>Hydrolyzed Collagen | - | - | - | - | 2,0 | - | 2,0 | - | - | - |
| **Gluadin® AGP**<br>Hydrolyzed Wheat Gluten | 0,5 | 0,5 | 0,5 | - | - | - | - | 0,5 | - | - |
| **Gluadin® WK**<br>Sodium Cocoyl Hydrolyzed Wheat Protein | 2,0 | 2,0 | 2,0 | 2,0 | 5,0 | - | - | - | 0,5 | 0,5 |
| **Euperlan® PK 3000 AM**<br>Glycol Distearate (and) Laureth-4 (and) Cocamidopropyl Betaine | 5,0 | - | - | 5,0 | - | - | - | - | - | - |
| **Arlypon® F**<br>Laureth-2 | - | - | - | - | - | - | - | - | - | - |
| **Panthenol-Mikrokapseln gemäß Bsp.3** | 1,0 | 1,0 | 1,0 | 1,0 | 1,0 | 1,0 | 1,0 | 1,0 | 1,0 | 1,0 |
| **Hydagen® CMF**<br>Chitosan | 1,0 | 1,0 | 1,0 | 1,0 | 1,0 | 1,0 | 1,0 | 1,0 | 1,0 | 1,0 |
| **Magnesium Sulfate Hepta Hydrate** | - | - | - | - | - | - | - | - | 1,0 | 1,0 |
| **Glycerin** (86 Gew.-%ig) | - | - | - | - | - | 3,0 | 3,0 | 5,0 | 5,0 | 3,0 |

(21-25) Schaumbad, (26) Softcreme, (27, 28) Feuchtigkeitsemulsion, (29, 30) Nachtcreme

**Tabelle 1**

**Beispiele für kosmetische Zubereitungen (Wasser, Konservierungsmittel ad 100 Gew.-%) – Forts.**

| **Zusammensetzung (INCI)** | **31** | **32** | **33** | **34** | **35** | **36** | **37** | **38** | **39** | **40** |
|---|---|---|---|---|---|---|---|---|---|---|
| **Dehymuls® PGPH**<br>Polyglyceryl-2 Dipolyhydroxystearate | 4,0 | 3,0 | - | 5,0 | - | - | - | - | - | - |
| **Lameform® TGI**<br>Polyglyceryl-3 Diisostearate | 2,0 | 1,0 | - | - | - | - | - | - | - | - |
| **Emulgade® PL 68/50**<br>Cetearyl Glucoside (and) Cetearyl Alcohol | - | - | - | - | 4,0 | - | - | - | 3,0 | - |
| **Eumulgin®B2**<br>Ceteareth-20 | - | - | - | - | - | - | - | 2,0 | - | - |
| **Tegocare® PS**<br>Polyglyceryl-3 Methylglucose Distearate | - | - | 3,0 | - | - | - | 4,0 | - | - | - |
| **Eumulgin VL 75**<br>Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS**<br>Glyceryl Stearate | - | - | - | - | - | 2,0 | 4,0 | - | - | 4,0 |
| **Lanette® O**<br>Cetearyl Alcohol | - | - | 2,0 | - | 2,0 | 4,0 | 2,0 | 4,0 | 4,0 | 1,0 |
| **Antaron® V 216**<br>PVP / Hexadecene Copolymer | - | - | - | - | - | 3,0 | - | - | - | 2,0 |
| **Myritol® 818**<br>Cocoglycerides | 5,0 | - | 10,0 | - | 8,0 | 6,0 | 6,0 | - | 5,0 | 5,0 |
| **Finsolv® TN**<br>C12/15 Alkyl Benzoate | - | 6,0 | - | 2,0 | - | - | 3,0 | - | - | 2,0 |
| **Cetiol® J 600**<br>Oleyl Erucate | 7,0 | 4,0 | 3,0 | 5,0 | 4,0 | 3,0 | 3,0 | - | 5,0 | 4,0 |
| **Cetiol® OE**<br>Dicaprylyl Ether | 3,0 | - | 6,0 | 8,0 | 6,0 | 5,0 | 4,0 | 3,0 | 4,0 | 6,0 |
| **Mineral Oil** | - | 4,0 | - | 4,0 | - | 2,0 | - | 1,0 | - | - |
| **Cetiol® PGL**<br>Hexadecanol (and) Hexyldecyl Laurate | - | 7,0 | 3,0 | 7,0 | 4,0 | - | - | - | 1,0 | - |
| **Bisabolol** | 1,2 | 1,2 | 1,2 | 1,2 | 1,2 | 1,2 | 1,2 | 1,2 | 1,2 | 1,2 |
| **Panthenol-Mikrokapseln gemäß Bsp.3** | 1,0 | 1,0 | 1,0 | 1,0 | 1,0 | 1,0 | 1,0 | 1,0 | 1,0 | 1,0 |
| **Hydagen® CMF**<br>Chitosan | 1,0 | 1,0 | 1,0 | 1,0 | 1,0 | 1,0 | 1,0 | 1,0 | 1,0 | 1,0 |
| **Copherol® F 1300**<br>Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro**<br>Sodium Phenylbenzimidazole Sulfonate | 3,0 | - | - | 3,0 | - | - | 2,0 | - | 2,0 | - |
| **Neo Heliopan® 303**<br>Octocrylene | - | 5,0 | - | - | - | 4,0 | 5,0 | - | - | 10,0 |
| **Neo Heliopan® BB**<br>Benzophenone-3 | 1,5 | - | - | 2,0 | 1,5 | - | - | - | 2,0 | - |
| **Neo Heliopan® E 1000**<br>Isoamyl p-Methoxycinnamate | 5,0 | - | 4,0 | - | 2,0 | 2,0 | 4,0 | 10,0 | - | - |
| **Neo Heliopan® AV**<br>Octyl Methoxycinnamate | 4,0 | - | 4,0 | 3,0 | 2,0 | 3,0 | 4,0 | - | 10,0 | 2,0 |
| **Uvinul® T 150**<br>Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

(31) W/O-Sonnenschutzcreme, (32-34) W/O-Sonnenschutzlotion, (35, 38, 40) O/W-Sonnenschutzlotion

## Patentansprüche

1. Mikrokapseln mit mittleren Durchmessern im Bereich von 0,1 bis 5 mm, bestehend aus einer Hüllmembran und einer mindestens einen Wirkstoff enthaltenden Matrix, dadurch erhältlich, dass man

   (a1) aus Gelbildnern, Chitosanen und Wirkstoffen eine Matrix herstellt,
   (a2) die Matrix in einer Ölphase dispergiert,
   (a3) zur Bildung der Hülle die dispergierte Matrix mit wässrigen Lösungen anionischer Tenside in Kontakt bringt, und
   (a4) die Ölphase wieder entfernt,

   oder

   (b1) aus Gelbildnern, anionischen Tensiden und Wirkstoffen eine Matrix herstellt,
   (b2) die Matrix in einer Ölphase dispergiert,
   (b3) zur Bildung der Hülle die dispergierte Matrix mit wässrigen Chitosanlösungen in Kontakt bringt, und
   (b4) die Ölphase wieder entfernt.

2. Verfahren zur Herstellung von Mikrokapseln mit mittleren Durchmessern im Bereich von 0,1 bis 5 mm, bestehend aus einer Hüllmembran und einer mindestens einen Wirkstoff enthaltenden Matrix, bei dem man

   (a1) aus Gelbildnern, Chitosanen und Wirkstoffen eine Matrix herstellt,
   (a2) die Matrix in einer Ölphase dispergiert,
   (a3) zur Bildung der Hülle die dispergierte Matrix mit wässrigen Lösungen anionischer Tenside in Kontakt bringt, und
   (a4) die Ölphase wieder entfernt,

   oder

   (b1) aus Gelbildnern, anionischen Tensiden und Wirkstoffen eine Matrix herstellt,
   (b2) die Matrix in einer Ölphase dispergiert,
   (b3) zur Bildung der Hülle die dispergierte Matrix mit wässrigen Chitosanlösungen in Kontakt bringt, und
   (b4) die Ölphase wieder entfernt.

3. Verfahren nach Anspruch 2, **dadurch gekennzeichnet, dass** man als Gelbildner Heteropolysaccharide oder Proteine einsetzt.

4. Verfahren nach Anspruch 3, **dadurch gekennzeichnet, dass** man als Heteropolysaccharide Agarosen, Agar-Agar, Pektine, Xanthane sowie deren Gemische einsetzt.

5. Verfahren nach Anspruch 3, **dadurch gekennzeichnet, dass** man als Proteine Gelatine einsetzt.

6. Verfahren nach mindestens einem der Ansprüche 2 bis 5, **dadurch gekennzeichnet, dass** man Chitosane einsetzt, die ein mittleres Molekulargewicht im Bereich von 10.000 bis 500.000 bzw. 800.000 bis 1.200.000 Dalton aufweisen.

7. Verfahren nach mindestens einem der Ansprüche 2 bis 6, **dadurch gekennzeichnet, dass** man kosmetische Wirkstoffe einsetzt, die ausgewählt sind aus der Gruppe, die gebildet wird von kosmetischen Ölen, Perlglanzwachsen, Stabilisatoren, biogenen Wirkstoffen, Deodorantien, Antitranspirantien, Antischuppenmitteln, UV-Lichtschutzfaktoren, Antioxidantien, Konservierungsmitteln, Insektenrepellentien, Selbstbräunern, Parfümölen, Aromen und Farbstoffen.

8. Verfahren nach mindestens einem der Ansprüche 2 bis 6, **dadurch gekennzeichnet, dass** man Flammschutz-Wirkstoffe einsetzt, die ausgewählt sind aus der Gruppe, die gebildet wird von Metalloxiden, organischen Haliden und organischen Phosphorverbindungen.

9. Verfahren nach mindestens einem der Ansprüche 2 bis 8, **dadurch gekennzeichnet, dass** man die Mikrokapseln - bezogen auf das Kapselgewicht - mit 0,1 bis 25 Gew.-% Wirkstoff belädt.

**10.** Verfahren nach mindestens einem der Ansprüche 2 bis 9, **dadurch gekennzeichnet, dass** man bei der Herstellung der Matrix nichtionische Emulgatoren und/oder Viskositätsregulatoren mitverwendet.

**11.** Verfahren nach mindestens einem der Ansprüche 2 bis 10, **dadurch gekennzeichnet, dass** man anionische Tenside einsetzt, die ausgewählt sind aus der Gruppe, die gebildet wird von Alkylbenzolsulfonaten, Alkyl(ether)sulfaten, Alkyl(ether)phosphaten und Seifen.

**12.** Verfahren nach mindestens einem der Ansprüche 2 bis 11, **dadurch gekennzeichnet, dass** man die Matrix bei Temperaturen im Bereich von 40 bis 100 °C herstellt.

**13.** Verfahren nach mindestens einem der Ansprüche 2 bis 12, **dadurch gekennzeichnet, dass** man die Matrix mit 0,1 bis 10 Gew.-%igen wässrigen Lösungen der anionischen Tenside bzw. Chitosanen in Kontakt bringt.

**14.** Verfahren nach mindestens einem der Ansprüche 2 bis 13, **dadurch gekennzeichnet, dass** man die Matrix bei Temperaturen im Bereich von 40 bis 100 °C mit den wässrigen Tensid- bzw. Chitosanlösungen in Kontakt bringt.

**15.** Verwendung von Mikrokapseln nach Anspruch 1 zur Herstellung von kosmetischen und/oder pharmazeutischen Zubereitungen.

**16.** Verwendung von Mikrokapseln nach Anspruch 1 zur Herstellung von Garnen, Fasern und textilen Flächengebilden.

Europäisches Patentamt

# EUROPÄISCHER RECHERCHENBERICHT

Nummer der Anmeldung
EP 01 10 7153

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | EP 1 077 060 A (PRIMACARE S A) 21. Februar 2001 (2001-02-21) * Ansprüche 2,8; Beispiele H1-H5; Tabelle 1 * | 1-16 | B01J13/02 |
| D,X | EP 1 064 911 A (PRIMACARE S A) 3. Januar 2001 (2001-01-03) * Ansprüche 1-7,19-22; Beispiele 1-9 * | 1-16 | |
| D,A | DE 197 12 978 A (HENKEL KGAA) 1. Oktober 1998 (1998-10-01) * das ganze Dokument * | 1-16 | |

RECHERCHIERTE SACHGEBIETE (Int.Cl.7)
B01J
A61K

Der vorliegende Recherchenbericht wurde für alle Patentansprüche erstellt

| Recherchenort | Abschlußdatum der Recherche | Prüfer |
|---|---|---|
| DEN HAAG | 16. August 2001 | Willsher, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 01 10 7153

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-08-2001

| Im Recherchenbericht angeführtes Patentdokument | Datum der Veröffentlichung | Mitglied(er) der Patentfamilie | Datum der Veröffentlichung |
|---|---|---|---|
| EP 1077060 A | 21-02-2001 | AU 6281300 A | 13-03-2001 |
| | | WO 0112135 A | 22-02-2001 |
| EP 1064911 A | 03-01-2001 | AU 5977300 A | 22-01-2001 |
| | | WO 0101926 A | 11-01-2001 |
| DE 19712978 A | 01-10-1998 | AU 7036598 A | 22-10-1998 |
| | | WO 9843609 A | 08-10-1998 |
| | | EP 0969807 A | 12-01-2000 |

EPO FORM P0461

Für nähere Einzelheiten zu diesem Anhang : siehe Amtsblatt des Europäischen Patentamts, Nr.12/82